(19) Europäisches Patentamt
European Patent Office
Office européen des brevets

(11) Numéro de publication: **0 194 221**
**B1**

(12) **FASCICULE DE BREVET EUROPÉEN**

(45) Date de publication du fascicule du brevet: **08.06.88**

(51) Int. Cl.⁴: **C 07 C 69/712**, C 07 C 67/31

(21) Numéro de dépôt: **86450004.6**

(22) Date de dépôt: **24.01.86**

(54) **Nouvelle méthode de fabrication de l'ester de p-chlorophénol de l'acide p-chlorophénoxyisobutyrique.**

(30) Priorité: **28.02.85 FR 8503094**

(43) Date de publication de la demande:
**10.09.86 Bulletin 86/37**

(45) Mention de la délivrance du brevet:
**08.06.88 Bulletin 88/23**

(84) Etats contractants désignés:
**BE CH DE GB IT LI LU NL**

(56) Documents cité:
**FR-A-2 382 892**
**US-A-2 508 334**

(73) Titulaire: **SOCIETE CORTIAL S.A., 7 rue de l'Armorique, F-75015 Paris (FR)**

(72) Inventeur: **Feniou, Claude, 5 Rue du Général Guillomat, F-33600 Pessac (FR)**
Inventeur: **Descas, Patrick Domaine de Mauville, Bat C 288 299 Rue Pasteur, F-33200 Bordeaux (FR)**

(74) Mandataire: **Tajan, Marie- Thérèse, LABORATOIRES SARGET Avenue du Président JF Kennedy, F-33701 Mérignac (FR)**

**EP 0 194 221 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

La présente invention concerne une nouvelle méthode de fabrication du dulofibrate, produit utile dans le traitement des hyperlipémies.

Le dulofibrate est l'ester de p-chlorophénol de l'acide p-chlorophénoxyisobutyrique. Son emploi thérapeutique est décrit dans le brevet français n° FR-A-2 382 892, déposé le 8 mars 1977, et publié le 6 octobre 1978.

On connait plusieurs méthodes de préparation des esters de l'acide p-chlorophénoxyisobutyrique ou acide clofibrique.

Les esters d'alcools de l'acide clofibrique peuvent être préparés à partir de l'acide clofibrique et de l'alcool correspondant en présence d'ions $H^+$ mais cette méthode ne s'applique pas aux esters de phénols et ne peut donc pas être envisagée pour la fabrication du dulofibrate.

Une autre méthode plus générale de prépararation des esters de l'acide clofibrique consiste à faire réagir un dérivé de l'acide clofibrique tel qu'un chlorure ou un anhydride d'acide et un dérivé hydroxylé en présence par exemple d'une amine organique tertiaire dans un solvant apolaire tel que le toluène ou le benzène. Cette méthode peut s'appliquer à la préparation du dulofibrate et a été décrite dans le brevet français FR-A-2 382 892. Une variante consiste à faire réagir l'acide clofibrique et le dérivé hydroxylé que l'on désire estérifier en présence de dicyclohexylcarbodiimide.

L'acide clofibrique est préparé de façon générale à partir du p-chlorophénol et de l'acétone en présence de soude et de chloroforme. (référence Kleeman A., Engel J. Pharmazeutische Wirkstoffe. Synthese. Patente. Anwendungen. Vol II., Ed. Georg Thieme Verlag, New-York, 1982).

Les méthodes de préparation des esters clofibriques sont donc relativement longues et nécessitent de nombreuses étapes. La présente invention permet la fabrication du dulofibrate en deux étapes à partir du chlorophénol sans qu'il soit nécessaire d'isoler le produit intermédiaire; elle est beaucoup moins coûteuse et beaucoup plus rapide que les méthodes classiques de fabrication des esters de l'acide clofibrique. Elle consiste à préparer un sel de p-chlorophénol, de préférence un sel alcalin, puis à faire réagir ce phénolate, sans l'isoler, avec un halogénure d'un acide halogénoisobutyrique, de préference le bromure de l'acide bromoisobutyrique, dans un solvant non polaire choisi dans le groupe contenant le benzène, le dioxanne, le xylène et le toluène, de manière à obtenir directement l'ester de p-chlorophénol de l'acide clofibrique. Nous indiquons ci-dessous le schéma réactionnel dans le cas où on utilise le bromure de l'acide bromoisobutyrique et le p-chlorophénolate de sodium.

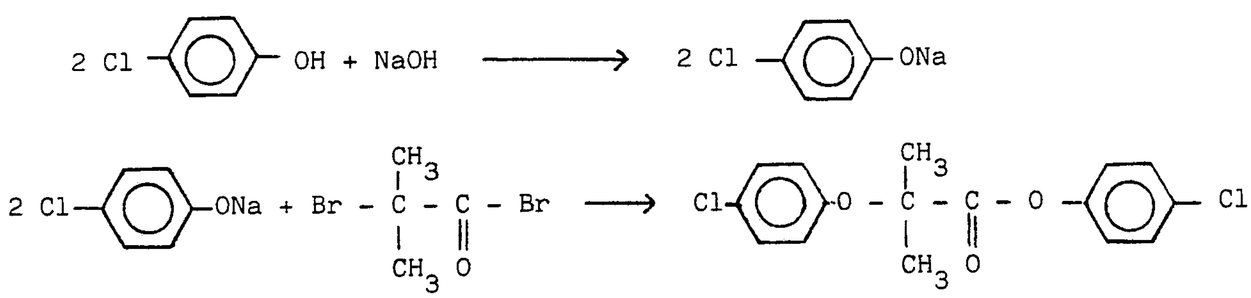

La méthode décrite dans la présente invention utilise comme réactif un halogénure d'acide halogénoisobutyrique. Un tel dérivé a été utilisé dans une méthode de préparation du simfibrate décrite dans le brevet japonais JP 7783422 (référence chemical abstracts 87 201117) au cours de laquelle le propanediol est estérifié par le bromure de bromoisobutyroyle, le dérivé dibromé ainsi obtenu réagissant avec le p-chlorophénolate de sodium pour donner le simfibrate. La méthode décrite dans la présente invention diffère de la méthode décrite dans le brevet japonais en ce sens que l'ester bromé intermédiaire n'est pas isolé et que l'on réalise au cours de la même étape l'estérification et la O-alkylation du p-chlorophénol.

La fabrication du sel alcalin de p-chlorophénol met en jeu une base telle que la soude ou la potasse, du p-chlorophénol et de l'eau. Le solvant apolaire est introduit dans le milieu réactionnel dès cette première étape, avant tout chauffage. Pour préparer 2 à 3 moles de p-chlorophénolate de sodium ou de potassium on utilise 2 à 3 moles de soude ou de potasse, 75 à 150 ml d'eau, 2 à 3 moles de p-chlorophénol et 1 à 2 l de solvant non polaire. On ajoute ensuite à la suspension contenant 2 à 3 moles de p-chlorophénolate de sodium ou de potassium dans un solvant apolaire, 1 mole d'halogénure d'acide halogénoisobutyrique. Le mélange réactionnel est maintenu sous agitation jusqu'à formation du bromo-2 méthyl-2 propanoate de p-chlorophényle puis est chauffé à des températures allant de 80 à 140°C, en fonction de la température d'ébullition du solvant, pendant 1 à 3 heures. Une variante consiste à introduire, avant de chauffer à 80 - 140°C, une quantité comprise entre 50 et 500 ml d'un solvant dipolaire aprotique choisi dans le groupe constitué par le diméthylformamide, l'acétone, le nitrométhane, le diméthylacétamide, le diméthylsulfoxyde, la tétraméthylurée et le dioxyde-1,1 de tétrahydrothiophène.

La présente invention va être décrite de façon plus précise dans les exemples suivants sans que ceux ci ne puissent restreindre sa portée.

**Exemple 1**

Dans un ballon de 1 litre sont introduits 20 ml d'eau, 20,4 g de soude à 98 % (0,5 mole) et 65 g de p-chlorophénol (0,5 mole). Le mélange réactionnel est maintenu sous agitation pendant 15 mn à température ambiante. 400 ml de toluène sont ajoutés. Le mélange est porté au reflux et l'eau formée au cours de la réaction est séparée dans une trappe de DEAN et STARCK.

Lorsque toute l'eau est séparée, le mélange est refroidi à température ambiante. 30 ml (0,242 mole) de bromure de l'acide bromo-2 méthyl-2 propionique sont ajoutés goutte à goutte en 30 mn. Le mélange réactionnel est maintenu sous agitation pendant 1 heure puis porté au reflux pendant 2 heures sous agitation vigoureuse à cause de la formation du précipité de bromure de sodium. La réaction est contrôlée par chromatographie sur couches minces de silice avec comme éluant le mélange 50/50 éther de pétrole/toluène. Les Rf du dulofibrate et du bromo-2 méthyl-2 propanoate de p-chlorophényle sont respectivement de 0,61 et 0,73.

Le mélange est refroidi à température ambiante. 250 ml d'eau sont ajoutés et le mélange est agité; la phase aqueuse est éliminée. 250 ml de soude 1N sont ajoutés et le mélange est agité; la phase aqueuse est éliminée. Cette dernière opération est renouvelée une fois. 250 ml d'eau sont ajoutés et le mélange est agité; la phase aqueuse est éliminée. Cette dernière opération est renouvelée jusqu'à neutralité de la dernière phase aqueuse éliminée. La phase organique est séchée sur chlorure de calcium. La solution obtenue après filtration est évaporée sous pression réduite.

50 ml d'éthanol à 95 % sont ajoutés. On laisse cristalliser sous agitation. Le dulofibrate est filtré et séché. On obtient ainsi 45,5 g de dulofibrate soit un rendement de 58 % par rapport au bromure de l'acide bromo-2 méthyl-2 propionique.

**Exemple 2**

On procède selon les modalités indiquées dans l'exemple 1 jusqu'à l'addition de 30 ml (0,242 mole) de bromure de l'acide bromo-2 méthyl-2 propionique. Le mélange réactionnel est maintenu sous agitation pendant 1 heure puis 50 ml de diméthylformamide sont introduits. Le mélange est chauffé à 115°C pendant 1 heure sous agitation vigoureuse. Le mélange est refroidi à température ambiante. 250 ml d'eau sont ajoutés et le mélange est agité; la phase aqueuse est éliminée. 250 ml de soude 1N sont ajoutés et le mélange est agité; la phase aqueuse est élimінее. Cette dernière opération est renouvelée une fois. 250 ml d'eau sont ajoutés et le mélange est agité; la phase aqueuse est éliminée. Cette dernière opération est renouvelée jusqu'à neutralité de la dernière phase aqueuse éliminée. La phase organique est séchée sur chlorure de calcium. La solution obtenue après filtration est évaporée sous pression réduite.

50 ml d'éthanol à 95 % sont ajoutés. On laisse cristalliser sous agitation. Le dulofibrate est filtré et séché.

**Revendications**

1. Méthode de préparation de l'ester de p-chlorophénol de l'acide p-chlorophenoxyisobutyrique caractérisée en ce que l'on transforme le p-chlorophénol en sel, de préférence alcalin, en présence d'un solvant non polaire choisi dans le groupe contenant le benzène, le dioxanne, le xylène et le toluène, puis en ce que l'on fait réagir 2 à 3 moles de ce p-chlorophénolate avec une mole d'un halogénure d'acide halogénoisobutyrique.

2. Méthode de préparation de l'ester de p-chlorophénol de l'acide p-chlorophenoxyisobutyrique selon la revendication 1 caractérisée en ce que l'on utilise le bromure de l'acide bromoisobutyrique.

3. Méthode de préparation de l'ester de p-chlorophénol de l'acide p-chlorophenoxyisobutyrique selon les revendications 1 et 2 caractérisée en ce que l'on prépare intermédiairement sans l'isoler le p-chlorophénolate de sodium à partir de p-chlorophénol et de soude dans l'eau en présence d'un solvant non polaire choisi dans le groupe contenant le benzène, le dioxanne, le xylène et le toluène, et en ce que l'on fait réagir ce p-chlorophénolate de sodium avec le bromure de l'acide bromoisobutyrique.

4. Méthode de préparation de l'ester de p-chlorophénol de l'acide p-chlorophenoxyisobutyrique selon les revendications 1 à 3 caractérisée en ce que l'on utilise, en tant que solvant apolaire, le toluène.

5. Méthode de préparation de l'ester de p-chlorophénol de l'acide p-chlorophenoxyisobutyrique selon les revendications 1 à 4 caractérisée en ce que l'on forme d'abord l'ester bromé intermédiaire dans le mélange réactionnel contenant le p-chlorophénolate, l'halogénure d'acide halogénoisobutyrique et le solvant apolaire, puis l'on soumet le mélange réactionnel à un chauffage à des températures allant de 80 à 140°C pendant 1 à 3 heures.

6. Méthode de préparation de l'ester de p-chlorophénol de l'acide p-chlorophenoxyisobutyrique selon les revendications 1 à 5 caractérisée en ce que l'on introduit dans le milieu réactionnel après formation de l'ester bromé intermédiaire, 50 à 500 ml d'un solvant dipolaire aprotique choisi dans le groupe constitué par le diméthylformamide, l'acétone, le nitrométhane, le diméthylacétamide, le diméthylsulfoxyde, la tétraméthylurée et le dioxyde-1,1 de tétrahydrothiophène pour 1 à 2 litres de solvant apolaire.

7. Méthode de préparation de l'ester de p-chlorophénol de l'acide p-chlorophenoxyisobutyrique selon la revendication 6 caractérisée en ce que l'on utilise en tant que solvant dipolaire aprotique le diméthylformamide.

**Patentansprüche**

1. Verfahren zur Herstellung des Esters des p-Chlorphenols mit p-Chlorphenoxyisobuttersäure, dadurch gekennzeichnet, daß man p-Chlorphenol in ein Salz, vorzugsweise ein Alkalisalz, in Gegenwart eines nicht-polaren Lösemittels aus der Benzol, Dioxan, Xylol und Toluol enthaltenden Gruppe überführt, und man 2 bis 3 Mol dieses p-Chlorphenolates mit einem Mol eines Säurehalogenids einer Halogeno-isobuttersäure umsetzt.

2. Verfahren zur Herstellung des Esters des p-Chlorphenols mit p-Chlorphenoxyisobuttersäure, dadurch gekennzeichnet, daß man das Säurebromid der Bromisobuttersäure einsetzt.

3. Verfahren zur Herstellung des Esters des p-Chlorphenols mit p-Chlorphenoxyisobuttersäure nach Anspruch 1 oder 2, dadurch gekennzeichnet, daß man intermediär ohne Isolierung das Natrium-p-chlorphenolat ausgehend von p-Chlorphenol und Natriumhydroxid in Wasser in Gegenwart eines nicht-polaren Lösemittels aus der Benzol, Dioxan, Xylol und Toluol enthaltenden Gruppe herstellt und das Natrium-p-chlorphenolat mit dem Säurebromid der Bromisobuttersäure umsetzt.

4. Verfahren zur Herstellung des Esters des p-Chlorphenols mit p-Chlorphenoxyisobuttersäure, nach einem der Ansprüche 1 bis 3, dadurch gekennzeichnet, daß man Toluol als apolares Lösemittel einsetzt.

5. Verfahren zur Herstellung des Esters des p-Chlorphenols mit p-Chlorphenoxyisobuttersäure nach einem der Ansprüche 1 bis 4, dadurch gekennzeichnet, daß man zunächst den Bromester als Zwischenstufe in dem das p-Chlorphenolat, das Säurehalogenid der Halogenoisobuttersäure und das apolare Lösemittel enthaltenden Reaktionsgemisch bildet, und man das Reaktionsgemisch 1 bis 3 Stunden einer Erwärmung auf Temperaturen von 80 bis 140° C unterwirft.

6.Verfahren zur Herstellung des Esters des p-Chlorphenols mit p-Chlorphenoxyisobuttersäure nach einem der Ansprüche 1 bis 5, dadurch gekennzeichnet, daß man in das Reaktionsgemisch nach der Bildung des intermediären Bromesters 50 bis 500 ml eines dipolaren, aprotischen Lösemittels aus der von Dimethylformamid, Aceton, Nitromethan, Dimethylacetamid, Dimethylsulfoxid, Tetramethylharnstoff und Tetrahydrothiophen-1,1-dioxid gebildeten Gruppe einführt.

7. Verfahren zur Herstellung des Esters des p-Chlorphenols mit p-Chlorphenoxyisobuttersäure nach Anspruch 6, dadurch gekennzeichnet, daß man als dipolares, aprotisches Lösemittel Dimethylformamid verwendet.

**Claims**

1. A process for preparing p-chlorophenol ester of p-chlorophenoxyisobutyric acid, characterized in that p-chlorophenol is transformed into a salt, preferably and alkaline salt, in the presence of a non-polar solvent, selected from the group comprising benzene, dioxane, xylene and toluene, then 2 to 3 moles of this p-chlorophenolate are reacted with one mole of a halogenoisobutyric acid halide.

2. A process for preparing p-chlorophenol ester of p-chlorophenoxyisobutyric acid according to claim 1, characterized in that bromoisobutyric acid bromide is used.

3. A process for preparing p-chlorophenol ester of p-chlorophenoxyisobutyric acid according to claims 1 and 2, characterized in that sodium p-chlorophenolate is prepared as an intermediate without being isolated from p-chlorophenol and sodium hydroxide in water in the presence of a non-polar solvent selected from the group comprising benzene, dioxane, xylene and toluene, and in that this sodium p-chlorophenolate is reacted with bromoisobutyric acid bromide.

4. A process for preparing p-chlorophenol ester of p-chlorophenoxyisobutyric acid according to claims 1 to 3, characterized in that toluene is used as apolar solvent.

5. A process for preparing p-chlorophenol ester of p-chlorophenoxyisobutyric acid according to claims 1 to 4, characterized in that first the intermediate brominated ester is formed in the reaction mixture containing the p-chlorophenolate, halogenoisobutyric acid halide and apolar solvent, then the reaction mixture is heated to temperatures in the range of 80 to 140° C for 1 to 3 hours.

6. A process for preparing p-chlorophenol ester of p-chlorophenoxyisobutyric acid according to claims 1 to 5, characterized in that after formation of the intermediate brominated ester, 50 to 500 ml of an aprotic dipolar solvent are added to the reaction medium, this solvent being selected from the group comprising dimethylformamide, acetone, nitromethane, dimethylacetamide, dimethylsulfoxide, tetramethylurea and tetrahydrothiophene 1,1-dioxide per 1 to 2 litres of apolar solvent.

7. A process for preparing p-chlorophenol ester of p-chlorophenoxyisobutyric acid according to claim 6, characterized in that dimethylformamide is used as aprotic dipolar solvent.